**Europäisches Patentamt**

**European Patent Office**

**Office européen des brevets**

(19)

(11) Veröffentlichungsnummer: **0 000 904**
**B1**

(12) **EUROPÄISCHE PATENTSCHRIFT**

(21) Anmeldenummer: **78100635.8**

(22) Anmeldetag: **09.08.78**

(51) Int. Cl³: **C 08 J 11/04, C 08 G 63/62**

(54) **Wiedergewinnung hochwertiger Polycarbonate aus Polycarbonat-Abfällen**

(30) Priorität: **20.08.77 DE 2737693**

(43) Veröffentlichungstag der Anmeldung:
**07.03.79 Patentblatt 79/05**

(45) Bekanntmachung des Hinweises auf die Patenterteilung:
**23.07.80 Patentblatt 80/15**

(84) Benannte Vertragsstaaten:
**DE FR NL**

(56) Entgegenhaltungen:
**DD - C - 45 575**
**DD - C - 45 599**
**DD - C - 45 600**
**DD - C - 46 282**
**DD - C - 46 353**
**DE - B - 1 234 026**
**DE - C - 910 593**
**FR - A - 2 283 766**
**Die Makromolekulare Chemie, 65, 1963, Seiten 252 - 253**

(73) Patentinhaber: **Bayer AG**
**Zentralbereich Patente, Marken und Lizenzen**
**D - 5090 Leverkusen 1, Bayerwerk (DE)**

(72) Erfinder: **Idel, Karsten, Dr.**
**Buyschstrasse 165**
**D - 4150 Krefeld (DE)**

Courier Press, Leamington Spa, England.

Weidergewinnung hochwertiger Polycarbonate aus Polycarbonat-Abfällen

Die vorliegende Erfindung betrifft ein Verfahren zur Rückgewinnung von aromatischen, hochmolekularen, thermoplastischen Polycarbonaten aus Polycarbonatabfällen, wobei die Polycarbonatabfälle sowohl rein als transparentes Naturmaterial als auch zusammen mit organischen und insbesondere anorganischen Farbstoffen und/oder anderen Additiven oder als Legierung im Verbund mit anderen thermoplastischen Materialien vorliegen können.

Aufgrund der fortschreitenden Verknappung von Rohstoffresourcen und den daraus resultierenden Rhostoffpreiserhöhungen werden Verfahren zur Aufarbeitung von nicht mehr verwendungsfähigen Kunststoffgebrauchsartikeln im Sinne einer Rückgewinnung entweder von Ausgangssubstanzen oder direkt des ursprünglichen Kunststoffes immer wichtiger.

So beschreibt E. Bullack in den Patentschriften DDR 45575, 46282, 45599, 45600 und 46353 Verfahren der Aufarbeitung von Polycarbonaten insbesondere im Hinblick auf die Gewinnung von 4,4'-Dioxy-diaryl-alkanen als Ausgangssubstanzen für die Polycarbonatsynthese.

Gemäß den Patentschriften DDR 45575, 46282 und 45599 werden die 4,4'-Dioxy-diarylalkane über Spaltung mittels Alkoholen, Säureanhydriden oder geringen Mengen basischer Katalysatoren nach z.T. aufwendigen Reinigungsverfahren erhalten. Gemäß den Patentschriften 45600 und 46353 wird die Spaltung durch Zugabe von Phenolen oder Diarylcarbonaten bei Anwesenheit von Metalloxid-Katalysatoren und Temperaturen über 180°C erreicht. Bei diesen Temperaturen besteht insbesondere bei Anwesenheit von Metalloxid-Katalysatoren die Gefahr von Nebenreaktionen, und umfangreiche Reinigungsoperationen sind zur Gewinnung sauberer Ausgangssubstanzen nötig.

Überraschenderweise wurde gefunden, daß man Polycarbonate sowohl in reiner oder eingefärbter Form als auch als Polymerlegierungen in einer glatt verlaufenden Eintopfreaktion schonend verseifen und gegebenenfalls nach Abfiltrieren von Additiven, Farbstoffen usw. oder Legierungsbestandteilen direkt anschließend in einer glatten Eintopfreaktion in gleichen Reaktionsmedien wieder zu hochmolekularen, thermoplastischen und löslichen Polycarbonaten aufphosgenieren kann. Ohne Zusatz später störender Katalysatoren und unter schonenden thermischen Bedingungen wurden somit Polycarbonate gespalten und das nach der Spaltung verbleibende Reaktionsgemisch ohne jede Zwischenreinigung direkt wieder zur Polycarbonatsynthese nach dem Phasengrenzflächenverfahren umgesetzt. Nach Zugabe eines nicht mit Wasser mischbaren, in der Phasengrenzflächenkondensation von Polycarbonaten gebräuchlichen Lösungsmittels wie z.B. Methylenechloride oder Chlorbenzol kann ohne jede Zwischenreinigung durch Einleiten von Phosgen direkt aufkondensiert werden. Der pH-Wert der alkalischen Phase sollte dabei je nach Art des Diphenols zwischen 9 und 14 gehalten werden. Üblicherweise werden vor und insbesondere nach der Phosgenierung tertiäre Amine wie z.B. Triäthylamin, Tributylamin, N-Alkylpiperidin oder Ammonium-, Sulfonium-, Phosphonium- oder Arsoniumverbindungen oder auch Stickstoffhetaryle wie beispielsweise Pyridin eingesetzt.

Die bei Polycarbonaten am Kettenende vorliegenden einwertigen Phenole befinden sich als Spaltprodukte nach dem Verseifungsschritt im alkalischen Reaktionsmedium und bauen während oder nach der Phosgenierung wieder vollständig als Kettenbegrenzer ein, so daß die resynthetisierten Polycarbonate die gleiche Lösungsviskosität wie die als Ausgangsmaterial umgesetzten Polycarbonatabfälle erreichen. Sollen andere Lösungsviskositäten eingestellt werden, so kann für eine gewünschte höhere Lösungsviskosität eine zusätzliche Menge an freiem Bisphenol und für eine niedrigere Lösungsviskosität eine zusätzliche Menge an einwertigem Phenol zudosiert werden.

Ebenso werden einkondensierte Verbindungen mit mehr als zwei kondensationsfähigen funktionellen Gruppen wie z.B. Trisoder Tetraphenole, die als Verzweiger in Polycarbonate eingebaut sind, bei der Resynthese der Polycarbonate wieder vollständig eingebaut. Eine Änderung der Verzweigerkonzentration im rückgewonnenen Polycarbonat bietet sich wieder durch Zugabe einer zusätzlichen Menge an Verzweiger oder an freiem Bisphenol zu dem Reaktionsmedium vor der Phosgenierung an.

Gegenstand der vorliegenden Erfindung ist somit ein Verfahren zur Rückgewinnung von aromatischen, hochmolekularen, thermoplastischen Polycarbonaten aus Polycarbonatabfällen durch Abbau und Repolykondensation, das dadurch gekennzeichnet ist, daß man die Polycarbonatabfälle in Substanz oder in Lösung bei Temperaturen zwischen 25°C und 220°C gegebenenfalls unter Druck verseift, von unverseifbaren Bestandteilen abtrennt und anschließend das Verseifungsgemisch ohne weitere Reinigungs- und Aufarbeitungsschritte nach den Methoden der Zweiphasengrenzflächenpolykondensation phosgeniert und polykondensiert.

Als Polycarbonatabfälle sind alle nicht mehr verwendbaren Polycarbonatkunststoffartikel, aber auch die bei der Herstellung und Verformung des Polycarbonatkunststoff anfallenden Rückstände, Abfallprodukte, Verschnitt etc. zu verstehen.

Die für die Aufarbeitung geeigneten Polycarbonatabfälle resultieren aus aromatischen Homopolycarbonaten und Copolycarbonaten, denen z.B. ein oder mehrere der folgenden Diphenole zugrunde liegen:
Hydrochinon
Resorcin
Dihydroxydiphenyle
Bis-(hydroxyphenyl)-alkane
Bis-(hydroxyphenyl)-cycloalkane
Bis-(hydroxyphenyl)-sulfide
Bis-(hydroxyphenyl)-äther
Bis-(hydroxyphenyl)-ketone
Bis-(hydroxyphenyl)-sulfoxide
Bis-(hydroxyphenyl)-sulfone
$\alpha,\alpha'$ - Bis - (hydroxyphenyl) - diisopropyl-benzole
sowie beispielsweise deren kernhalogenierte Verbindungen. Diese und weitere geeignete Diphenole sind z.B. in der US-Patentschrift 3 028 365, und in der Monographie "H. Schnell, Chemistry and Physics of Polycarbonates, Interscience Publishers, New York, 1964" beschrieben.

Bevorzugte Diphenole sind z.B.:
4,4'-Dihydroxydiphenyl
2,2 - Bis - (4 - hydroxyphenyl) - propan (Bisphenol - A)
2,4-Bis-(4-hydroxyphenyl)-2-methylbutan
1,1-Bis-(4-hydroxyphenyl)-cyclohexan
$\alpha,\alpha'$-Bis-(4-hydroxyphenyl)-p-diisopropylbenzol
2,2-Bis-(3-chlor-4-hydroxyphenyl)-propan
2,2-Bis-(3,5-dichlor-4-hydroxyphenyl)-propan
2,2-Bis-(3,5-dibrom-4-hydroxyphenyl)-propan.

Die als Polycarbonatabfälle aufarbeitbaren aromatischen Polycarbonate können auch durch den Einbau geringer Mengen, vorzugsweise von Mengen zwischen 0,05 und 2,0 Mol-% (bezogen auf eingesetzte Diphenole), an drei- oder mehr als drei-funktionellen Verbindungen, insbesondere solchen mit drei oder mehr als drei phenolischen Hydroxygruppen verzweigt sein, beispielsweise durch den Einbau von Phloroglucin, 1,3,5-Tri-(4-hydroxy-phenyl)-benzol, 1,1,1-Tri-(4-hydroxyphenyl)-äthan oder 1,4-Bis-(4,4'-dihydroxytriphenyl-methyl)-benzol.

Die als Polycarbonatabfälle aufarbeitbaren aromatischen Polycarbonate haben in der Regel mittlere Gewichtsmittelmolekulargewichte $\overline{M}w$ von 10 000 bis über 200 000, vorzugsweise von 20 000 bis 80 000, ermittelt durch Messungen der rel. Viskosität in $CH_2Cl_2$ bei 25°C und einer Konzentration von 0,5 Gew.-%.

Die Molekulargewichte der Polycarbonate können in üblicher Weise geregelt sein, beispielsweise durch den Einbau von Phenol, Tribromphenol oder p-tert.-Butylphenol.

Die als Polycarbonatabfälle aufarbeitbaren Polycarbonate können auch als Gemische von verschiedenen Polycarbonaten, beispielsweise mit Anteilen niedermolekularer Polycarbonate vermischt, vorliegen, oder auch als Polymerlegierungen beispielsweise mit Polymerisaten oder Copolymerisaten auf Basis Styrol, Styrol-Acrylnitril, Acrylnitril-Butadien-Styrol oder Butadien-Kautschuk vorliegen.

Die Verseifung der Polycarbonatabfälle erfolgt in Substanz oder in Lösung, wobei als Lösungsmittel die üblichen, für die Polycarbonatherstellung nach dem Zweiphasengrenzflächenverfahren geeigneten Lösungsmittel wie Methylenchlorid oder Chlorbenzol verwendet werden.

Die Verseifung erfolgt in alkalischem wäßrigem Reaktionsmedium, wobei die Verseifung in Substanz heterogen und die Verseifung in Lösung an der Grenzfläche des Zweiphasensystems erfolgt, wobei letztere günstiger und rascher verläuft. Bezüglich des wäßrigen Reaktionsmediums werden vorzugsweise zwischen 25 und 100 Mol Wasser pro Mol zu verseifender Polycarbonat-Struktureinheit eingesetzt.

Al bassische Verseifungsagenzien dienen z.B. Natriumhydroxid, Kaliumhydroxid, Calciumoxid, vorzugsweise Natriumhydroxid.

Die Verseifung erfolgt bei Temperaturen zwischen 25° und 220°C und gegebenenfalls unter Verwendung von Überdruck bis vorzugsweise 100 Atmosphären.

Die Menge an basischen Verseifungsagenzien liegt zwischen der 0,01 und 15-fachen Molmenge, bezogen auf die Mole entstehender aromatischer Dihydroxyverbindung; der pH-Wert des Verseifungsmediums liegt zwischen 9 und 14; die Dauer der Verseifung je nach sterischer Abschirmung der Carbonatstruktur zwischen 0,5 und max. 20 Stunden.

Es können als Katalysatoren zusätzlich Phosphite oder Phosphine zugesetz werden.

Enthalten die Polycarbonatabfälle unverseifbare Bestandteile wie Gleitmittel, Stabilisatoren, Pigmente, Farbstoffe, Füllstoffe wie Glaspulver, Quarzerzeugnisse, Graphit, Molybdänsulfid, Metallpulver, Pulver höherschmelzender Kunststoffe wie Polytetraäthylenpulver, natürliche Fasern wie Asbest, ferner Glasfasern der verschiedensten Art, Metallfäden etc., so werden diese nach erfolgter Filtration auf bekannte Weise, beispielsweise durch Filtration abgetrennt.

Die nach der Verseifung der Polycarbonatabfälle resultierenden ein- oder zweiphasigen Lösungen können direkt für die Polycarbonatherstellung nach dem Phasengrenzflächenverfahren eingesetzt werden, wobei gegebenenfalls die gebräuchlichen Polycarbonat-Lösungsmittel wie Methylenchlorid oder Chlorbenzol zugesetzt werden und der pH-Wert der wäßrigen Lösung je nach Anforderung zwischen 9 und 14 eingestellt wird.

Die nachfolgende Phosgenierung erfolgt gewöhnlich bei Raumtemperatur, wobei vorzugsweise eine Phosgenmenge von 1 bis 3 Mol, bezogen auf 1 Mol Diphenol, eingesetzt wird.

Die Dauer der Phosgenierung beträgt max. 1 Stunde.

Tertiäre Amine oder andere Polykondensationskatalysatoren werden in Mengen zwischen 0,01 und 10 Mol-%, bezogen auf die Mole Diphenole, vor oder insbesondere nach der Phosgenierung zugesetzt.

Die Polykondensation erfolgt anschließend in der üblichen Weise bei Temperaturen zwischen 20° und 40°C und ist je nach eingesetztem Diphenol nach einem Zeitraum von 1—5 Std. beendet.

Die erhaltenen Polycarbonatlösungen werden wie üblich aufgearbeitet und das Polycarbonat in der üblichen Weise isoliert.

### BEISPIEL 1

25,4 g Polycarbonat aus 2,2-Bis-(4-hydroxyphenyl)-propan mit einer Lösungsviskosität von $\eta_{rel} = 1,288$ (gemessen an Lösungen von 0,5 g Polycarbonat in 100 ml Methylenchlorid) werden in Granulatform in 80 ml 15 n Natronlauge gegeben. Das Reaktionsgemisch wird 4 Std. auf 100°C erhitzt. In dieser Zeit wird eine vollständige Spaltung des Polycarbonats bis zum Monomerbaustein Bisphenol A erreicht. Gibt man 500 ml dest. Wasser zu der resultierenden Suspension, so erhält man eine klare alkalische Lösung des Bisphenol A, die nach Zugabe von 500 ml Methylenchlorid nach den üblichen Methoden der Phasengrenzflächenkondensation phosgeniert werden kann.

Hierzu werden in 1 Stunde bei 25°C 15 g Phosgen unter Einstellung eines pH-Wertes von 13—14 eingeleitet und anschließend wird nach Zugabe von 1,5 ml einer 4 %igen wäßrigen Triäthylaminlösung noch 1 h aufkondensiert. Die organische Phase wird abgetrennt, zweimal mit 2 %iger Phosphorsäure und dann mit dest. Wasser elektrolytfrei gewaschen. Nach dem Abdampfen des Lösungsmittels erhält man 24 g Polycarbonat mit einer $\eta_{rel} = 1,295$. Das recyclisierte Polycarbonat zeigt die gleichen guten mechan. und rheologischen Eigenschaften wie das Ausgangsmaterial.

### BEISPIEL 2

25,4 g Polycarbonat aus 2,2-Bis-(4-hydroxyphenyl)-propan des Beispiels 1 werden in Granulatform in 30 ml 15 n Natronlauge gegeben. Zusätzlich werden noch 2,5 g (10 Gew.-%) Triphenylphosphit zugesetzt und dann gemäß Beispiel 1 bei 100°C verseift. Durch Zusatz des Phosphits verkürzt sich die Zeit bis zur vollständigen Verseifung auf 2 Std. 45 min. Danach wird analog Beispiel 1 in der Phasengrenzfläche aufphosgeniert. Das zurückgewonnene Polycarbonat besitzt eine Lösungsviskosität von $\eta_{rel} = 1,297$ und zeigt nach Ausfällen aus der Methylenchloridlösung mittels Methanol in den mechanischen und rheologischen Eigenschaften keinen Unterschied zum Ausgangsmaterial.

### BEISPIEL 3

25,4 g Polycarbonat aus 2,2-Bis-(4-hydroxyphenyl)-propan gemäß Beispiel 1 werden in 300 ml Chlorbenzol gelöst und 80 ml 15 n Natronlauge als alkalische Phase hinzugegeben. Das Gemisch wurde auf 100°C gebracht und die organische Phase in kurzen Abständen auf den Gehalt an noch unverseiftem Polycarbonat geprüft. Bereits nach 60 Min. war die Spaltung vollständig abgeschlossen und das Phasengrenzflächengemisch konnte direkt nach Zugabe von 350 ml Wasser analog Beispiel 1 phosgeniert und aufgearbeitet werden. Man erhält 23,5 g eines Polycarbonats mit einer $\eta_{rel}$ bon 1,315 und ähnlichem Eigenschaftsbild wie das Ausgansmaterial.

Durch Zugabe von 450 mg p-tert.-Butylphenol als zusätzlichen Kettenabbrecher zum Verseifungsgemisch kann nach Phosgenierung und Aufkondensation die Lösungsviskosität von $\eta_{rel} = 1,282$ eingestellt werden.

### BEISPIEL 4

Verseifungsdauer und Menge der eingesetzten Base stehen in einem direkten Verhältnis. Wie die untenstehende Tabelle zeigt, kann die eingesetzte Menge Natronlauge auf Kosten verlängerter Verseifungszeiten reduziert werden. Die Verseifung wurde in der Grenzfläche durchgeführt: 25,4 g Polycarbonat des Beispiels 1 wurden in 300 ml Chlorbenzol gelöst und mit wechselnden Mengen 15 n Natronlauge versetzt. Es wurde jeweils die Zeit bis zur vollständigen Verseifung des Polycarbonats gemessen:

25,4 g Polycarbonat des Beispiels 1

| in 300 ml Chlorobenzol | + 80 ml 15 n NaOH | 55 min. Verseifungszeit |
|---|---|---|
| ,, | + 63 ml ,, | 70 ,, ,, |
| ,, | + 46 ml ,, | 120 ,, ,, |
| ,, | + 33 ml ,, | 260 ,, ,, |

### BEISPIEL 5

Die Verseifung kann auch unter Druck durchgeführt werden. 101,6 g Polycarbonat des Beispiels 1, 400 ml dest. Wasser und 2,26 g 45 %ige Natronlauge werden in einem Autoklaven in 1 h auf 210°C bei 50 bar Druck erhitzt und 2 Std. dabei gehalten. Das erhaltene Gemisch wird mit 840 g 6,2 %iger Natronlauge und 1500 ml Methylenchlorid versetzt. Unter Einhaltung eines pH-Wertes von 13—14 werden 60 g Phosgen in 1 h eingeleitet und anschließend nach Zugabe von 6 ml 4 %igem wäßrigen Triäthylamin 1 h aufkondensiert. Das Reaktionsgemisch wird gemäß Beispiel 1 aufgearbeitet. Da bei der Druckverseifung vermutlich geringe Mengen monofunktioneller Monomerer abgespalten werden, beträgt die Lösungsviskosität des resultierenden Polycar-

bonats $\eta_{rel} = 1,212$. Durch zusätzliche Zugabe von 19 g. Bisphenol A zum Verseifungsgemisch und entsprechende Erhöhung der Anteile Phosgen und Triäthylamin kann ein Polycarbonat der gewünschten $\eta_{rel} = 1,287$ erhalten werden.

## BEISPIEL 6

27,9 g eines Polycarbonats auf Basis Bisphenol A ($\eta_{rel} = 1,305$) mit 10 Gew.-% Glasfaser wird mit 80 ml 15 n Natronlauge versetzt und das Gemisch auf 100°C erwärmt. Nach 3 h ist die Verseifung beendet. Man gibt 500 ml dest. $H_2O$ zu dem Verseifungsmisch und filtriert die verbleibende Glasfaser ab. Das Filtrat wird mit 500 ml Methylenchlorid versetzt und analog Beispiel 1 mit 15 g Phosgen versetzt und 1,5 ml einer 4 %igen wäßr. Triäthylaminlösung aufkondensiert. Nach der Aufarbeitung gemäß Beispiel 1 erhält man 10 g eines Glasfaserfreien Polycarbonats $\eta_{rel} = 1,317$.

Das Polycarbonat zeigt das gleiche Eigenschaftsbild wie ein Polycarbonat entsprechender Kettenlänge, das durch übliche Kondensation von Bisphenol A und Phosgen nach dem Phasengrenzflächenverfahren gewonnen wurde.

## BEISPIEL 7

25,4 g eines Polycarbonats auf Basis Bisphenol A mit einer Lösungsviskosität von $\eta_{rel} = 1,367$, das 0,2 Mol-%, bezogen auf die Anteile Diphenole, des tetrafunktionellen Verzweigers 1,4-Bis-(4,4'-Dihydroxytriphenylmethyl)-benzol eingebaut enthält, wird mit 80 ml 15 n Natronlauge versetzt und 3 Std. bei 100°C gehalten. Danach gibt man 500 ml dest. $H_2O$ bis zur klaren Lösung und anschließend 500 ml Methylenchlorid hinzu. Das Verseifungsgemisch wird analog Beispiel 1 mit 15 g Phosgen versetzt und mit 1,5 ml einter 4 %igen wäßrigen Triäthylaminlösung aufkondensiert. Nach den üblichen Aufarbeitungsmethoden erhält man ein Polycarbonat mit einer Lösungsviskosität von $\eta_{rel} = 1,375$, das nach Spaltung und chromatographischer Untersuchung der Monomerbestandteile 0,2 Mol-% eines tetrafunktionellen Verzweigers eingebaut enthält.

## BEISPIEL 8

36,3 g einer Polymerlegierung, die sich aus 30 Gew.-% eines ABS-Polymeren und 70% eines Polycarbonats auf Basis Bisphenol A ($\eta$rel. 1.292) zusammensetzt, werden mit 120 ml 15 n Natronlauge 10 Std. bei 100°C behandelt. Danach füllt man mit 500 ml dest. $H_2O$ auf und filtriert das ungelöste ABS-Polymerisat ab. Anschließend gibt man noch 500 ml Methylenchlorid zu dem Filtrat und leitet in 1 h bei Raumtemperatur 11 g Phosgen ein und kondensiert nach Zugabe von 1,5 ml einer 4 %igen wäßrigen Triäthylaminlösung noch 1 h auf. Man erhält nach den üblichen Aufarbeitungsmethoden 17 g des hochmolekularen, thermoplastischen Polycarbonats mit einer Lösungsviskosität $\eta_{rel} = 1,305$.

## BEISPIEL 9

25,4 g eines mit 0,35 Gew.-% Cadmiumselenid rot eingefärbten Polycarbonats auf Basis Bisphenol A ($\eta_{rel}$ % 1,285) wird mit 80 ml Natronlauge versetzt und 3 Std. auf 100°C erwärmt. Danach wird mit 500 ml dest. $H_2O$ aufgefüllt und das ungelöste Farbpigment abfiltriert. Zu dem klaren Filtrat werden 500 ml Methylenchlorid gegeben und das Phasengrenzflächengemisch wie in Beispiel 1 phosgeniert und aufgearbeitet. Es verbleiben 22 g eines ungefärbten, thermoplast. Polycarbonats mit einer Lösungsviskostät von $\eta_{rel} = 1,297$.

## BEISPIEL 10

27 g eines Copolycarbonats auf Basis Bisphenol A und Tetrabrom-Bisphenol A (5,3 Gew.-% Brom) ($\eta_{rel} = 1,297$) werden in 150 ml Chlorbenzol gelöst und 80 ml 15 n Natronlauge zudosiert. Nach 4 Std. wird die Verseifung beendet und man erhält nach Zugabe von 350 ml Methylenchlorid und 400 ml Wasser ein klares Phasengemisch. Zur Rückgewinnung des Polycarbonats werden 15 g Phosgen in 1 h bei Raumtemperatur eingeleitet. Am Ende der Phosgenierung sollte die Lösung einen pH-Wert von ca. 11—12 haben. Nach Zugabe von 0,43 ml Triäthylamin kondensiert man noch 1 h auf und arbeitet gemäß Beispiel 1 auf. Es resultiert ein bromhaltiges Polycarbonat (5,4 % Brom) mit einer Lösungsviskosität von $\eta_{rel} = 1,285$ und ähnlichen Eigenschaften wie das Ausgangsmaterial.

## Patentanspruch

Verfahren zur Rückgewinnung aromatischer, hochmolekularer, thermoplastischer Polycarbonate aus Polycarbonatabfällen durch Abbau und Repolykondensation, dadurch gekennzeichnet, daß man die Polycarbonatabfälle in Substanz oder in Lösung bei Temperaturen zwischen 25°C und 220°C gegebenenfalls unter Druck verseift, von unverseifbaren Bestandteilen abtrennt, und anschließend das Verseifungsgemisch ohne weitere Reinigungs- und Aufarbeitungsschritte nach den Methoden der Zweiphasengrenzflächenpolykondensation phosgeniert und polykondensiert.

## Revendication

Procédé pour récupérer des polycarbonates aromatiques macromoléculaires thermoplastiques à partir de déchets de polycarbonates par dégradation et repolycondensation, ce procédé se caractérisant en ce que l'on saponifie les déchets de polycarbonates tels quels ou en solution à des températures de 25 à 220°C, éventuellement sous pression, on sépare les constituants non saponifiables et on soumet ensuite le mélange de saponification, sans autre opération de purification ni d'isolement, à phosgénation et polycondensation par les techniques de polycondensation à l'interface de deux phases.

## Claim

A process for the recovery of aromatic, high molecular weight, thermoplastic polycarbonates from polycarbonate scrap by means of decomposition and repolycondensation, characterised in that the polycarbonate scrap is saponified in bulk or in solution at temperatures between 25°C and 220°C, optionally under pressure, the non-saponified constituents are separated off and the saponification mixture is then phosgenated and subjected to polycondensation according to the methods of two phase boundary polycondensation without any further purification steps and treatment steps.